# EUROPEAN PATENT SPECIFICATION

(11) **EP 3 015 125 B1**
(45) Date of publication and mention of the grant of the patent: **07.11.2018**
(21) Application number: 15185512.9
(22) Date of filing: 16.09.2015
(51) Int. Cl.: A61M 1/36

(54) **SYSTEMS FOR COLLECTING MONONUCLEAR CELLS**
SYSTEME ZUM SAMMELN VON EINKERNIGEN ZELLEN
SYSTÈMES SERVANT À RECUEILLIR DES CELLULES MONONUCLÉAIRES

(30) Priority: 27.10.2014 US 201414524115
(43) Date of publication of application: 04.05.2016
(73) Proprietor: Fenwal, Inc., Lake Zurich, IL 60047 (US)
(72) Inventor: Radwanski, Katherine N., Lake Zurich, IL 60047 (US)
(74) Representative: Nollen, Maarten Dirk-Johan

(56) References cited:
- EP-A1- 2 641 623
- EP-A2- 1 066 842
- WO-A1-99/01197
- WO-A1-03/015850
- US-A1- 2004 127 841
- ANDREU G ET AL: "Extracorporeal photochemotherapy: Evaluation of two techniques and use in connective tissue disorders", TRANSFUSION SCIENCE, PERGAMON PRESS, OXFORD, GB, vol. 15, no. 4, 1 December 1994 (1994-12-01), pages 443-454, XP026405249, ISSN: 0955-3886 [retrieved on 1994-12-01]

## Description

### Field of the Invention

The present disclosure is directed to systems and methods for collecting mononuclear cells. More particularly, the present invention is directed to systems for collecting mononuclear cells with a reduced volume of residual plasma so as to reduce plasma interference during photopheresis.

### Background

Whole blood is made up of various cellular components such as red cells, white cells (or leukocytes) and platelets suspended in its liquid component, plasma. Whole blood can be separated into its constituent components (cellular or liquid), and the desired component can be separated so that it can be administered to a patient in need of that particular component. For example, mononuclear cells (MNCs), primarily lymphocytes and monocytes, can be removed from the whole blood of a patient, collected, and subjected to photodynamic therapy in a procedure commonly referred to as extracorporeal photopheresis, or ECP. In ECP, MNCs are treated with a photosensitizing agent and subsequently irradiated with specified wavelengths of light to achieve a desired effect, and returned to the patient for the treatment of various blood diseases to, e.g., eliminate immunogenicity in cells, inactivate or kill selected cells, inactivate viruses or bacteria, or activate desirable immune responses.

More specifically, separated MNCs may be separated from whole blood, chemically treated with a light-activated agent, such as 8-methoxypsoralen (8-MOP, either added to the separated MNCs or administered in advance to the patient), exposed to ultraviolet light, and returned to the patient. The activated 8-methoxypsoralen crosslinks with the DNA in the exposed MNCs, ultimately resulting in apoptosis of the MNCs. The photochemically-damaged MNCs returned to the patient to induce cytotoxic effects on T-cell formation, resulting in an antitumor action.

Commonly, MNCs are collected by introducing whole blood into a centrifuge chamber wherein the whole blood is separated into its constituent components based on the size and densities of the different components, with the targeted component(s) being collected, and the non-target components either being returned to the patient or otherwise disposed of.

Typical blood processing systems thus include a permanent, reusable centrifuge assembly containing the hardware (drive system, pumps, valve actuators, programmable controller, and the like) that spins and pumps the blood, and a disposable, sealed and sterile fluid processing assembly that is mounted cooperatively on the hardware. The centrifuge assembly spins a disposable centrifuge chamber in the fluid processing assembly during a collection procedure, thereby separating the blood into its constituent components.

A difficulty in performing phototherapy is the delivery of the proper dose of light energy to the photoactivatable material in the suspension, particularly if the suspension includes material that is not substantially transparent to light so that it attenuates the light energy intended for photoactivation. Specifically, during the harvest of MNCs for photopheresis, other non-target substances are present in the MNC product that can interfere with delivering the intended dose of UV-A light to the target MNCs. Red blood cells absorb the majority of UV-A light in MNCs products. However, plasma proteins and lipids can also contribute to UV-A light absorption, especially in the case of certain disease states or medications, such as elevated bilirubin levels and drugs such as mycophenolate mofetil (MMF) and cyclosporine, which cause hyperlipidemia.

EP1066842 A2 (published on 10.1.2001) relates to phototherapy and photopheresis systems where an effective amount of light energy is desired to be delivered to targets in biological fluids.

By way of the present disclosure, systems and methods are provided for collecting mononuclear cells with a reduced volume of residual plasma so as to reduce plasma interference during photopheresis.

### Summary of the invention

The invention is defined by the claims. In one aspect, not forming part of the invention, the present disclosure is directed to a method for obtaining mononuclear cells and preparing the mononuclear cells for photopheresis with a separation device. The method comprises: separating mononuclear cells from a biological fluid that includes red blood cells, plasma and platelets and collecting a targeted number of mononuclear cells in a suspension including plasma and residual red blood cells and platelets; concentrating the separated mononuclear cells; removing plasma from the concentrated mononuclear cells until the amount of residual plasma remaining with the concentrated mononuclear cells reaches a pre-determined volume; and adding a crystalloid solution to the concentrated mononuclear cells.

In another aspect of the method, not forming part of the invention, the volume of residual plasma remaining with the concentrated mononuclear cells may be less than or equal to (≤) 50 mL.

In another aspect, not forming part of the invention, the concentrated mononuclear cells may have a hematocrit of less than or equal to (≤) 2%, after adding the crystalloid solution.

In a further aspect of the method, not forming part of the invention, crystalloid solution may be added to the concentrated mononuclear cells in an amount so that the volume % of plasma is less than or equal to (≤) 25%.

In a related aspect, not forming part of the invention, the amount of crystalloid solution may be added to the suspension in an amount of from 150 mL to 200mL.

In another aspect of the method, not forming part of the invention, the separation device may be flushed with crystalloid solution or plasma.

In one aspect, the invention is directed to a system for the collection of mononuclear cells as defined in claim 1. The system includes a reusable hardware apparatus with a separation device and a programmable microprocessor driven controller including instructions for processing a biological fluid. The system also includes a disposable processing circuit associated with the apparatus, wherein the circuit includes a processing chamber for receiving a biological fluid and one or more containers for collecting mononuclear cells. The controller is programmed to separate mononuclear cells from a biological fluid that includes red blood cells, plasma and platelets, concentrate the mononuclear cells and remove plasma until the residual plasma remaining with the concentrated mononuclear cells reaches a pre-determined volume, and combine the concentrated mononuclear cells with a crystalloid solution such that the volume% of plasma is less than or equal to 25 %.

In another aspect, the present invention is directed to a mononuclear cell product as defined in claim 7 that includes mononuclear cells, plasma, crystalloid solution and residual red blood cells and platelets having a total volume of less than or equal to (≤) 200mL and a hematocrit of less than or equal to (≤) 2%. The product has a volume % of plasma of less than or equal to (≤) 25 %.

In a preferred embodiment, the volume of residual plasma remaining with the concentrated mononuclear cells is less than or equal to 50 mL.

### Brief Description of the Drawings

Fig. 1 is a is a perspective view of an automated apheresis device that may be used in the collection and other processing steps of mononuclear cells in accordance with the present disclosure;
Fig. 2 is an enlarged perspective view of the front panel of the device of Fig. 3 with an exemplary disposable processing set for collecting mononuclear cells mounted on the device;
Fig. 3 is a diagram showing the disposable processing set of Fig. 2;
Fig. 4 is a graph showing lymphocyte apoptosis levels during 72 hours of culture post ECP treatment (30 J/cm²) of MNC products (200 mL, 2% Hct) suspended in predominantly plasma or saline solution; and
Fig. 5 is a graph showing standard curves generated from purified MNCs in saline treated with known UV-A light doses ranging from 0 - 2.0 J/cm².

### Detailed Description of the Embodiments

A more detailed description of the systems and methods in accordance with the present disclosure is set forth below. It should be understood that the description below of specific devices and methods is intended to be exemplary, and not exhaustive of all possible variations or applications. Thus, the scope of the disclosure is not intended to be limiting, and should be understood to encompass variations or embodiments that would occur to persons of ordinary skill.

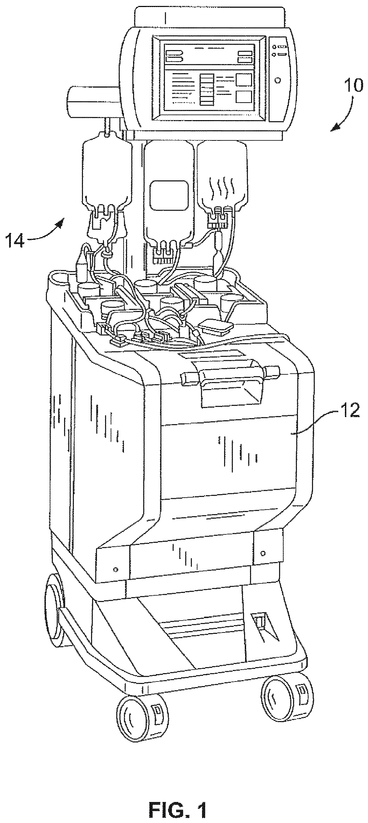

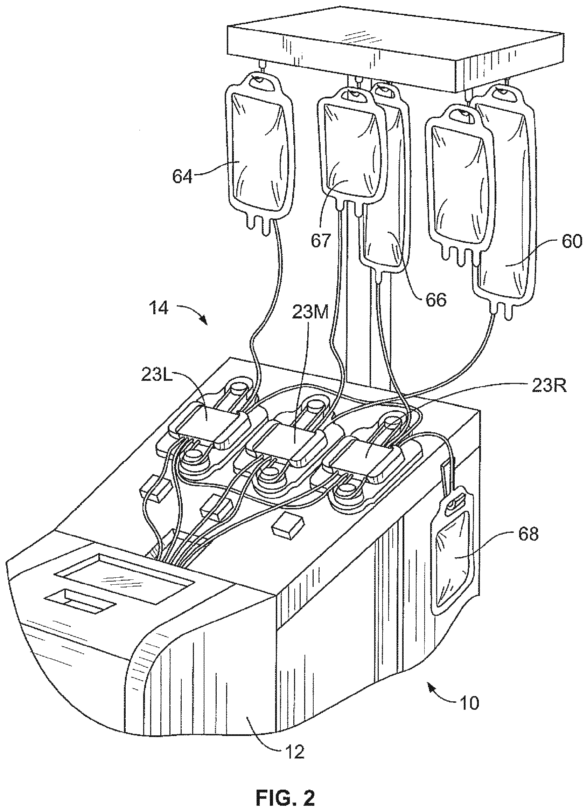

Figs. 1 and 2 show a representative separation system 10 useful in the separation and collection of mononuclear cells, as described herein. The system 10 includes a durable separation component 12 and a disposable processing kit 14 that is mounted thereon. In one embodiment, the separation principle used by the separator 12 is based on centrifugation, but an automated separator based on a different separation principle may also be used.

When used to perform photopheresis, an irradiation component housed separately from separation component is used in conjunction with the separation system 10, as shown and described in US 2013/0197419. Although separately housed and independent devices, it is preferable that separation device and irradiation device be located adjacent to each other. However, it will be appreciated that the methods described herein may also be used with devices having integrated separation and irradiation components.

In accordance with the systems and methods described herein a patient is connected to a blood processing set, i.e., fluid circuit 14. The fluid circuit 14 provides a sterile closed pathway between the separation component 12 and the irradiation component. The system described herein also optionally includes a washing component which, preferably, is housed within the separation component 12. Preferably, the separation component 12 and washing component are one and the same.

With reference to Fig. 1, whole blood is withdrawn from the patient and introduced into the separation component 12, where the whole blood is separated to provide a target cell population, which in the context of the present disclosure may be mononuclear cells. Other components separated from the whole blood, such as red blood cells and platelets may be returned to the patient or collected in pre-attached containers of the blood processing set. The separated target cell population, e.g., mononuclear cells, is then prepared for treatment in accordance with the methods described in greater detail below.

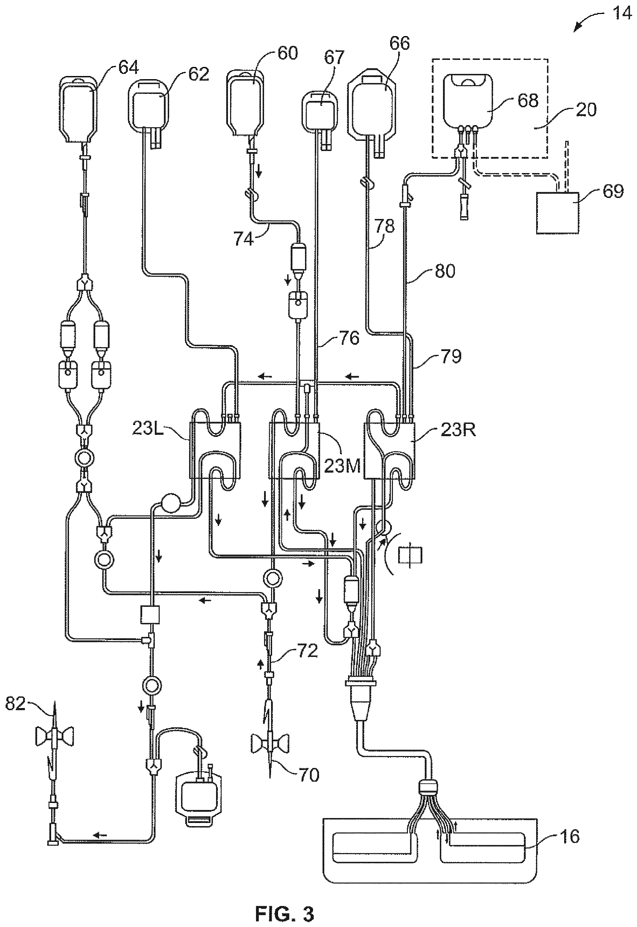

Apparatus useful in the collection (and washing) of mononuclear cells, and providing the separation component 12 of Fig. 1, include the Amicus® Separator made and sold by Fenwal, Inc., of Lake Zurich, Illinois. Mononuclear cell collections using a device such as the Amicus® are described in greater detail in U.S. Patent No. 6,027,657. The fluid circuit (Fig. 3) includes a blood processing container 16 defining a separation chamber suitable for harvesting mononuclear cells (MNC) from whole blood.

As shown in Fig. 2, a disposable processing set or fluid circuit 14 (which includes container 16) is mounted on the front panel of the separation component 12. The processing set (fluid circuit 14) includes a plurality of processing fluid flow cassettes 23L, 23M and 23R with tubing loops for association with peristaltic pumps on separation component 12. Fluid circuit 14 also includes a network of tubing and pre-connected containers for establishing flow communication with the patient and for processing and collecting fluids and blood and blood components, as shown in greater detail in Fig. 3.

As seen in Fig. 3, the disposable processing set 14 may include a container 60 for supplying anticoagulant, a waste container 62 for collecting waste from one or more steps in the process for treating and washing mononuclear cells, a container 64 for holding a crystalloid solution, such as saline, or other wash or resuspension medium, a container 66 for collecting plasma, a container 68 for collecting the mononuclear cells and, optionally, container 69 for holding photoactivation agent.

Container 68 may also serve as the illumination container, and is preferably pre-attached to with the disposable set 14. Alternatively, container 68 may be attached to set 14 by known sterile connection techniques, such as sterile docking or the like.

With reference to Fig. 3, fluid circuit includes inlet line 72, an anticoagulant (AC) line 74 for delivering AC from container 60, an RBC line 76 for conveying red blood cells from chamber 12 of container 14 to container 67, a platelet-poor plasma (PPP) line 78 for conveying PPP to container 66 and line 80 for conveying mononuclear cells to and from separation chamber 16 and collection/illumination container 68.

The blood processing set also includes one or more venipuncture needle(s) for accessing the circulatory system of the patient. As shown in Fig. 3, fluid circuit 14 includes inlet needle 70 and return needle 82. In an alternative embodiment, a single needle can serve as both the inlet and outlet needle.

Fluid flow through fluid circuit 14 is preferably driven, controlled and adjusted by a microprocessor-based controller in cooperation with the valves, pumps, weight scales and sensors of separation component 12 and fluid circuit 14, the details of which are described in the previously mentioned U.S. Patent No. 6,027,657.

The illustrated fluid circuit is further adapted for association with the treatment component (i.e., irradiation device). Apparatus for the irradiation of the mononuclear cells are also known and are available from sources such as Cerus Corporation, of Concord, California. One example of a suitable irradiation device is described in U.S. Patent No. 7,433,030. As shown and described in U.S. Patent No. 7,433,030, irradiation device preferably includes a tray or other holder for receiving one or more containers during treatment. Other irradiation devices may also be suitable for use with the method and system described herein, including devices available from Macopharma and/or Vilber Lourmet.

A separation chamber is defined by the walls of the processing container 16. In operation, the separation device 12 rotates the processing container 16 about an axis, creating a centrifugal field within the processing container 16. Details of the mechanism for causing of the processing container 16 are disclosed in U.S. Patent No. 5,360,542 entitled "Centrifuge with Separable Bowl and Spool Elements Providing Access to the Separation Chamber."

In one embodiment, an apheresis device may include a programmable controller that is pre-programmed with one or more selectable protocols. A user/operator may select a particular processing protocol to achieve a desired outcome or objective. The pre-programmed selectable protocol(s) may be based on one or more fixed and/or adjustable parameters. During a particular processing procedure, the pre-programmed controller may operate the centrifuge and processing chamber associated therewith to separate blood into its various components, as well as operate one or more pumps to move blood, blood components and/or solutions through the various openable valves and tubing segments of a processing set, such as such as processing set 14 illustrated in Fig. 3. This may include, for example, initiating and causing the centrifugal separation of mononuclear cells from whole blood in the separation chamber 16, removing plasma from mononuclear cells (i.e., pumping the removed plasma to a storage or waste bag) to obtain MNC concentrate, purging or flushing the tubing segments to collect additional MNCs that may reside or remain in the tubing during or after processing, and combining a crystalloid solution to the concentrated MNCs. The various processing steps performed by the pre-programmed automated apheresis device may occur separately, in series, simultaneously or any combination of these.

In accordance with the present disclosure, an automated apheresis device may be used to perform MNC collection in a batch process in which MNCs continuously collect in the chamber 16 until the target cycle volume is reached. As the MNCs are transferred out of the chamber 16, they pass through an optical sensor which detects the presence of cells in the tubing line to determine the start and end of the MNC harvest (i.e. when to open and close the valves leading to the product container). After MNC harvest is complete, the remaining cells in the line are flushed into the product container with a predetermined volume of plasma known as the "plasma flush". The plasma volume could be further reduced by reducing, eliminating or replacing the plasma flush with a crystalloid solution flush.

The volume of the MNC product (which contains MNCs, RBCs and platelets in plasma) is fixed at a low predetermined value. If needed, additional cycle(s) of WB processing and MNC harvest can be performed to achieve the desired yield of MNCs to be treated. Most of the plasma is removed from the MNC concentrate to arrive at a predetermined volume of residual plasma, and the MNC concentrate is then reconstituted with a crystalloid solution to obtain a MNC product ready for phototherapy. More specifically, sufficient crystalloid solution (preferably saline) is added to the concentrated MNCs to obtain the desired hematocrit for UV-A irradiation (typically around 150 -200 mL of saline is added to achieve a hematocrit of 2% or less). As the plasma content in the overall product represents typically 25% or less of the total volume, the effect of elevated plasma protein or lipid levels on UV-A dose delivered to MNCs is reduced.

Experimentation was performed to confirm the efficacy of the present method in reducing plasma interference during ECP. This was done by comparing lymphocyte apoptosis levels 72 hours after UV-A exposure for ECP-treated MNC products suspended in plasma versus ECP-treated MNC products suspended in a crystalloid solution (i.e., saline).

MNC products were collected from healthy donors using the following collection settings: 1 cycle, WB/cycle = 2000 mL, plasma flush = 10 mL, and plasma storage fluid = 150 mL or 0 mL. MNC products were suspended in approximately 220 mL volume in order to provide 20 mL samples for study controls (+ 8MOP, no UV). For MNC products collected with 150 mL plasma storage fluid, approximately 30 mL of saline was added to the product post collection using a sterile syringe (plasma arm, n = 6). For MNC products collected with 0 mL storage fluid, approximately 190 mL of saline was added to the product post collection using a sterile syringe (saline arm, n = 6).

8-MOP was added to each MNC product to a final concentration of 200 ng/mL and products (200 mL, ∼2 % Hct) were irradiated with 30 J/cm²UV-A light (320 - 400 nm) with an intensity of 13-20 J/cm². After irradiation, MNCs were purified using a Ficoll-Paque gradient, washed twice with RPMI 1640 media, and re-suspended at 1-2 x 10⁶/mL in RPMI 1640 media supplemented with 2 mM glutamine and 10% human serum.

Cells were cultured at 37°C in a humidified chamber with 5% CO₂ for up to 72 hours. After 24, 48 and 72 hours, samples were assayed for apoptosis. Lymphocyte apoptosis was measured as the percentage of CD45+/Annexin-V positive cells in the lymphocyte forward/side scatter gate.

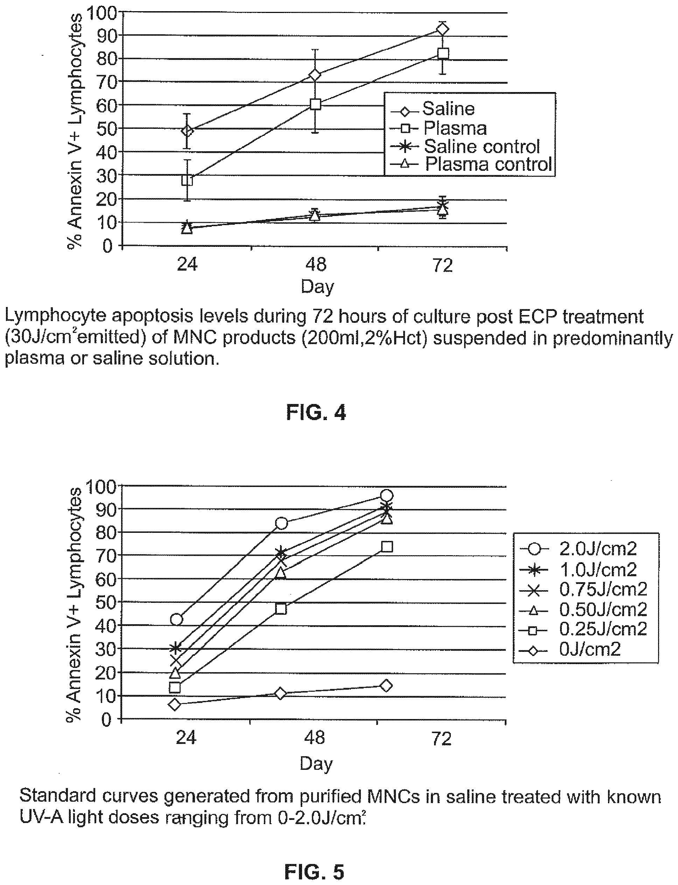

The results are plotted in Figs. 4 and 5. During culture, treated lymphocytes in the plasma arm of the study exhibited an apoptotic response similar to 0.5 - 1.0 J/cm² delivered to the MNCs, while the saline arm was similar to 1.5 - 2.0 J/cm². It was thus concluded that using primarily saline solution in the MNC product versus plasma resulted in at least 2 times greater UV-A light dose delivered to the MNCs.

### Examples

Without limiting any of the foregoing, the subject matter described herein may be found in one or more methods, systems and/or products. For example, in a first aspect of the present disclosure a method, not forming part of the invention, for obtaining MNCs is set forth. The method includes: separating mononuclear cells from a biological fluid that includes red blood cells, plasma and platelets and collecting a targeted number of mononuclear cells in a suspension including plasma and residual red blood cells and platelets; concentrating the separated mononuclear cells; removing plasma from the concentrated mononuclear cells until the amount of residual plasma remaining with the concentrated mononuclear cells reaches a pre-determined volume; and adding a crystalloid solution to the concentrated mononuclear cells. In another aspect of the method, the volume of residual plasma remaining with the concentrated mononuclear cells may be less than or equal to (≤) 50 mL.

In another aspect, the concentrated mononuclear cells may have a hematocrit of less than or equal to (≤) 2%, after adding the crystalloid solution.

In a further aspect of the method, crystalloid solution may be added to the concentrated mononuclear cells in an amount so that the volume % of plasma is less than or equal to (≤) 25%.

In a related aspect, the amount of crystalloid solution may be added to the suspension in an amount of from 150 mL to 200mL.

In another aspect of the method, the separation device may be flushed with crystalloid solution or plasma.

In another aspect, the present disclosure is directed to a system for the collection of mononuclear cells. The system includes a reusable hardware apparatus with a separation device and a programmable microprocessor driven controller including instructions for processing a biological fluid. The system also includes a disposable processing circuit associated with the apparatus, wherein the circuit includes a processing chamber for receiving a biological fluid and one or more containers for collecting mononuclear cells. The controller is programmed to separate mononuclear cells from a biological fluid that includes red blood cells, plasma and platelets, concentrate the mononuclear cells and remove plasma until the residual plasma remaining with the concentrated mononuclear cells reaches a pre-determined volume, and combine the concentrated mononuclear cells with a crystalloid solution.

In another aspect, the present disclosure is directed to a mononuclear cell product that includes mononuclear cells, plasma, crystalloid solution and residual red blood cells and platelets having a total volume of less than or equal to (≤) 200mL and a hematocrit of less than or equal to (≤) 2%. In a related aspect, the product may have a volume % of plasma of less than or equal to (≤) 25% and/or a volume of residual plasma of less than or equal to (≤) 50 mL.

## Claims

1. A system for the collection of mononuclear cells comprising:
a) a reusable hardware apparatus, said apparatus including a separation device preferably comprising a rotatable element that generates a centrifugal field sufficient to separate mononuclear cells from other components of a biological fluid and a programmable microprocessor driven controller including instructions for processing a biological fluid;
b) a disposable processing circuit associated with said apparatus, said circuit including a separation chamber for receiving whole blood and one or more containers for collecting mononuclear cells;
c) wherein said controller is programmed to:
i. separate whole blood into a plurality of components including mononuclear cells, red blood cells, plasma and platelets in the separation chamber;
ii. concentrate said obtained mononuclear cells in the separation chamber and remove plasma from the concentrated mononuclear cells until an amount of residual plasma remaining with the concentrated mononuclear cells reaches a pre-determined volume; and
iii. deliver a selected volume of a crystalloid solution to the concentrated mononuclear cells in said residual amount of plasma, such that the volume% of plasma is less than or equal to (≤) 25%.

2. The system of Claim 1, wherein the controller is configured to concentrate said mononuclear cells such that a volume of residual plasma remaining with said concentrated mononuclear cells is less than or equal to (≤) 50 ml.

3. The system of Claim 1 or Claim 2, wherein the selected volume of the crystalloid solution is controlled such that the concentrated mononuclear cells has a hematocrit value of less than or equal to (≤) 2% after adding the crystalloid solution.

4. The system of any of the preceding claims, wherein the controller is configured for flushing the separation device with one of a crystalloid solution and plasma.

5. The system of any of the preceding claims, wherein the controller is further programmed to collect, after the separation step and before the concentration step, a targeted number of mononuclear cells in a suspension including plasma and residual red blood cells and platelets and wherein the targeted number of mononuclear cells is collected in a container suitable for irradiation of the mononuclear cells.

6. The system of Claim 1-5 further comprising a source of a crystalloid solution.

7. A mononuclear cell product comprising mononuclear cells, plasma, crystalloid solution and residual red blood cells and platelets having a total volume of less than or equal to (≤) 200mL and a hematocrit of less than or equal to (≤) 2%, **characterized in that** the volume % of plasma is less than or equal to (≤) 25%.

8. The mononuclear cell product of claim 7 wherein said volume of residual plasma remaining with said concentrated mononuclear cells is less than or equal to (≤) 50 ml.

## Patentansprüche

1. System zum Sammeln von einkernigen Zellen, das aufweist:
a) eine wiederverwendbare Hardwarevorrichtung, wobei die Vorrichtung eine Trennvorrichtung umfasst, die vorzugsweise aufweist: ein drehbares Element, das ein Zentrifugalfeld erzeugt, das ausreicht, um einkernige Zellen von anderen Komponenten eines biologischen Fluids zu trennen, und eine durch einen programmierbaren Mikroprozessor betriebene Steuerung, die Anweisungen zur Verarbeitung eines biologischen Fluids umfasst;
b) einen Wegwerf-Verarbeitungskreislauf, die mit der genannten Vorrichtung verbunden ist, wobei der Kreislauf eine Trennkammer zum Aufnehmen von Vollblutzellen und einen oder mehrere Behälter zum Sammeln von einkernigen Zellen umfasst;
c) wobei die Steuerung programmiert ist, um:
i. Vollblut in der Trennkammer in mehrere Komponenten zu trennen, die einkernige Zellen, rote Blutzellen, Plasma und Blutblättchen umfassen;
ii. die erhaltenen einkernigen Zellen in der Trennkammer zu konzentrieren und Plasma von den konzentrierten einkernigen Zellen zu entfernen, bis eine Menge an Restplasma, die in den konzentrierten einkernigen Zellen verbleit, ein vorgegebenes Volumen erreicht; und
iii. ein ausgewähltes Volumen einer kristalloiden Lösung an die konzentrierten einkernigen Zellen in der Restmenge von Plasma zuzuführen, so dass die Volumen-% des Plasmas kleiner oder gleich (≤) 25% sind.

2. System nach Anspruch 1, wobei die Steuerung konfiguriert ist, um die einkernigen Zellen zu konzentrieren, so dass ein Volumen von Restplasma, das bei den konzentrierten einkernigen Zellen verbleibt, kleiner oder gleich (≤) 50 ml ist.

3. System nach Anspruch 1 oder Anspruch 2, wobei das ausgewählte Volumen der kristalloiden Lösung derart gesteuert wird, dass die konzentrierten einkernigen Zellen einen Hämatokritwert von kleiner oder gleich (≤) 2% haben, nachdem die kristalloide Lösung zugegeben wurde.

4. System nach einem der vorhergehenden Ansprüche, wobei die Steuerung konfiguriert ist, um die Trennvorrichtung mit einer kristalloiden Lösung oder Plasma zu spülen.

5. System nach einem der vorhergehenden Ansprüche, wobei die Steuerung ferner programmiert ist, um nach den Trennungsschritt und vor dem Konzentrationsschritt eine geplante Anzahl einkerniger Zellen in einer Suspension, die Plasma und Restblutzellen und Blutblättchen enthält, zu sammeln, und wobei die geplante Anzahl einkerniger Zellen in einem Behälter gesammelt wird, der für die Bestrahlung der einkernigen Zellen geeignet ist.

6. System nach Anspruch 1 - 5, das ferner eine Quelle einer kristalloiden Lösung aufweist.

7. Einkerniges Zellprodukt, das einkernige Zellen, Plasma, kristalloide Lösung und Restblutzellen und Blutblättchen mit einem Gesamtvolumen kleiner oder gleich (≤) 200 ml und einen Hämatokritwert kleiner oder gleich (≤) 2% hat, **dadurch gekennzeichnet, dass** die Volumen-% des Plasmas kleiner oder gleich (≤) 25% sind.

8. Einkerniges Zellprodukt nach Anspruch 7, wobei das Volumen des Restplasmas, das bei den konzentrierten einkernigen Zellen verbleibt, kleiner oder gleich (≤) 50 ml ist.

## Revendications

1. Système servant à recueillir des cellules mononucléées comprenant :
a) un appareil matériel réutilisable, ledit appareil comprenant un dispositif de séparation comprenant de préférence un élément pivotant qui produit un champ centrifuge suffisant pour séparer des cellules mononucléées des autres composants d'un liquide biologique et un contrôleur dirigé par un microprocesseur programmable comprenant des instructions pour traiter un liquide biologique ;
b) un circuit de traitement jetable associé audit appareil, ledit circuit comprenant une chambre de séparation pour la réception du sang total et un ou plusieurs récipients pour recueillir les cellules mononucléées ;
c) dans lequel ledit contrôleur est programmé pour :
i. séparer le sang total en une pluralité de composants comprenant les cellules mononucléées, les érythrocytes, le plasma et les plaquettes dans la chambre de séparation ;
ii. concentrer lesdites cellules mononucléées obtenues dans la chambre de séparation et éliminer le plasma des cellules mononucléées concentrées jusqu'à ce qu'une quantité de plasma résiduel demeurant avec les cellules mononucléées concentrées atteigne un volume prédéterminé ; et
iii. administrer un volume choisi d'une solution cristalloïde aux cellules mononucléées concentrées dans ladite quantité résiduelle de plasma, de telle façon que le volume en % du plasma est inférieur ou égal à (≤) 25 %.

2. Système selon la revendication 1, dans lequel le contrôleur est configuré pour concentrer lesdites cellules mononucléées de telle façon qu'un volume de plasma résiduel demeurant avec lesdites cellules mononucléées concentrées est inférieur ou égal (≤) 50 ml.

3. Système selon la revendication 1 ou la revendication 2, dans lequel le volume choisi de la solution cristalloïde est contrôlé de telle façon que les cellules mononucléées concentrées ont une valeur d'hématocrite inférieure ou égale à (≤) 2 % après l'ajout de la solution cristalloïde.

4. Système selon l'une quelconque des revendications précédentes, dans lequel le contrôleur est configuré pour rincer le dispositif de séparation avec l'un d'une solution cristalloïde et du plasma.

5. Système selon l'une quelconque des revendications précédentes, dans lequel le contrôleur est en outre programmé pour recueillir, après l'étape de séparation et avant l'étape de concentration, un nombre ciblé de cellules mononucléées dans une suspension comprenant du plasma et des érythrocytes et des plaquettes résiduels et dans lequel le nombre ciblé de cellules mononucléées est recueilli dans un récipient approprié pour l'irradiation des cellules mononucléées.

6. Système selon les revendications 1 à 5 comprenant en outre une source de solution cristalloïde.

7. Produit de cellules mononucléées comprenant des cellules mononucléées, du plasma, de la solution cristalloïde et des érythrocytes et des plaquettes résiduels ayant un volume total inférieur ou égal à (≤) 200 ml et un hématocrite inférieur ou égal à (≤) 2 %, **caractérisé en ce que** le volume en % du plasma est inférieur ou égal à (≤) 25 %.

8. Produit de cellules mononucléées selon la revendication 7 dans lequel ledit volume du plasma résiduel demeurant avec lesdites cellules mononucléées concentrées est inférieur ou égal à (≤) 50 ml.
